Europäisches Patentamt

European Patent Office

Office européen des brevets

(19)

(11) Publication number: **0 220 797**
**A2**

# (12) EUROPEAN PATENT APPLICATION

(21) Application number: 86305780.8

(22) Date of filing: 28.07.86

(51) Int. Cl.⁴: **A61K 9/50** , B01J 13/02

(30) Priority: 21.10.85 JP 234779/85

(43) Date of publication of application:
06.05.87 Bulletin 87/19

(84) Designated Contracting States:
CH DE FR GB IT LI

(71) Applicant: NIKKO CHEMICALS CO. LTD.
4-8, Nihonbashi-Bakurocho, 1-chome
Chuo-Ku Tokyo(JP)
Applicant: NIHON SURFACTANT KOGYO K.K.
24-3, Hasune 3-chome
Itabashi-ku Tokyo(JP)

(72) Inventor: Matumoto, Koichi c/o Nikko
Chemicals Co. Ltd.
4-8, Nihonbashi-Bakurocho, 1-chome
Chuo-ku Tokyo(JP)
Inventor: Shinozaki, Kazuhiko
Nihon Surfactant Kogyo K.K. 24-3, Hasune
3-chome
Itabashi-ku Tokyo(JP)
Inventor: Tabata, Yujin
Nihon Surfactant Kogyo K.K. 24-3, Hasune
3-chome
Itabashi-ku Tokyo(JP)

(74) Representative: Taylor, Phillip Kenneth et al
W.P. THOMPSON & CO. Coopers Building
Church Street
Liverpool L1 3AB(GB)

(54) Process for the preparation of liposome.

(57) A process for the preparation of a liposome which comprises mixing water with both a phospholipid and a hydrophilic nonionic surfactant. The water is preferably concurrently mixed with both the surfactant and the phospholipid. The phospholipid may be a hydrogenated phospholipid.

EP 0 220 797 A2

## PROCESS FOR THE PREPARATION OF LIPOSOME

The present invention relates to a process for the preparation of a liposome.

A liposome is used in various fields such as foods or cosmetics as well as medicines. For example, the envelopment of a drug in a liposome brings about various effects such as the delivery of a drug to an application region in the body, the acceleration of the absorption of a drug, enhancement in the persistency of the effects of a drug, the stabilization of a drug or the like. Further, in the fields of cosmetics, a humectant is enveloped in a liposome to thereby enable the percutaneous absorption thereof or an unstable component is stabilized by utilizing a liposome.

Many processes for the preparation of a liposome have been known, among which ultrasonic treatment method (thin film method), injection method or surfactant-treatment method is mainly used.

The ultrasonic treatment method comprises adding an aqueous solution to a thin phospholipid film, mechanically vibrating the obtained mixture with a vortex mixer or the like to obtain a multilamellar liposome (MLV) and subjecting the MLV to ultrasonic treatment with a sonicator at a temperature higher than the phase transition temperature (Tc) to obtain a small single compartment liposome (SUV).

The injection method comprises injecting a solution of phospholipid in ethanol into an aqueous solution by the use of a microsyringe under vigorous stirring to prepare a SUV.

The surfactant-treatment method comprises dissolving phospholipid in an aqueous solution of sodium cholate and subjecting the obtained solution to gel filtration or dialysis to remove the sodium cholate. This method is thought to mainly give a large single compartment liposome (LUV).

However, these methods are not suitable for the mass-production, thus being not practical.

Namely, the vortex method or the ultrasonic method requires the preformation of a dry thin phospholipid film, so that the area of the film is small for the volume of the device. Thus, these methods are very unsuitable for the industrial mass-production of a liposome.

The ethanol injection method is advantageous in that the operation is relatively easy and that the enlargement in the scale is possible to a certain extent, but is disadvantageous in that the ethanol can not be removed completely, so that no uniform liposomes can be formed and the ethanol remains in the final formation and that the extent of envelopment is low. Further, the surfactant-treatment method is disadvantageous in that the removal of sodium cholate is troublesome and that the preparation thereof is difficult in the presence of an acid or heavy metal salt, because the cholate is an anionic surfactant.

Consequently, a process for the preparation of a liposome which does not require any complicated device and can afford a fine liposome in a high productivity for a short time has long been desired.

It has now been surprisingly found that a minute liposome can be easily prepared by using both phospholipid and a hydrophilic nonionic surfactant. The present invention has been accomplished on the basis of this finding.

The present invention provides a process for the preparation of a liposome, which comprises mixing water, preferably concurrently, with both a phospholipid and a hydrophilic nonionic surfactant. The fact that a liposome having a small particle size can be easily formed by utilizing a hydrophilic nonionic surfactant is a surprise, because a nonionic surfactant was previously thought to destroy a liposome.

The hydrophilic nonionic surfactant to be used in the present invention is preferably soluble in water completely. Therefore it is preferred that the nonionic surfactant used has a hydrophile lipophile balance - (HLB) of 12 or above. Preferred examples of the nonionic surfactant include polyoxyethylene alkyl ethers, polyoxyethylene fatty acid esters, polyoxyethylene hydrogenated castor oil, polyoxyethylene vegetable oil, polyoxyethylene glycerin fatty acid esters, polyoxyethylene propylene glycol fatty acid esters, polyoxyethylene sorbitol (sorbitan) fatty acid esters, sucrose fatty acid esters, polyglycerin fatty acid esters, polyoxyethylene glycol ether and polyoxypropylene glycol ether.

Examples of the phospholipid to be used in the present invention include animal and vegetable lecithins such as egg yolk lecithin and soybean lecithin and purified and hydrogenated derivatives thereof.

Further, a dispersing agent such as polyhydric alcohol, organic or inorganic salt or water-soluble polymer may be used when dispersing phospholipid and a hydrophilic nonionic surfactant into water.

Furthermore, a liposome stabilizer such as cholesterol, soybean oil, castor oil, arginine, casein, glycerin monostearate or glycerin distearate can be also used.

The amount of the hydrophilic surfactant used is preferably 1 to 15% by weight based on the weight of the phospholipid used. If it exceeds 15% by weight, the formed liposome may be destroyed. When it is desired to envelope any desired component such as a drug in a liposome, the required amount of the desired component may be present during the formation of the liposome in the manner of the present invention or may be added after the formation of the liposome, the former being preferred.

When the desired component is a water-soluble one, a W/O emulsion containing the desired component in the inner aqueous phase thereof may be formed, followed by the addition of phospholipid and a nonionic surfactant to obtain a liposome containing the W/O emulsion. Thus a liposome containing various components such as drugs can be prepared.

According to the present invention, a minute liposome having a particle size of 0.1 to 0.3 micron. can be obtained by using a small amount of a hydrophilic nonionic surfactant. Further, a liposome can be prepared in a high productivity for a short time with an ordinary device without the use of a sonicator or the like, so that the cost of the preparation thereof is reduced to thereby enable the use of liposome in more fields.

Now, the present invention will be further described by referring to the following Examples, though it is not limited by them.

Example 1

A mixture having a formulation which will be described below was homogeneously dissolved and spray-dried to remove the hexane, thus obtaining a powdery sample. 5 g of this sample was added to 100 cc of water. The obtained mixture was stirred by the use of a homomixer at a speed of 8000 rpm for one minute to obtain a liposome liquid.

The obtained liposome had an average particle size of 0.2 micron and was stable.

Formulation:

| purified soybean lecithin | 8 g |
|---|---|
| cholesterol | 2 |
| soybean oil | 1 |
| tocopherol | 1 |
| hexane | 50 |
| octaethylene glycol dodecyl ether | 1 |

Example 2

A mixture having a formulation which will be described below was stirred under heating to obtain a homogeneous solution. 100 g of warm water was added to the obtained solution at 80°C while stirring the solution at a speed of 5000 rpm. The obtained mixture was rapidly cooled to obtain a liposome. This liposome was a minute particle having an average size of 0.3 micron and was stable.

Formulation:

| purified hydrogenated soybean lecithin | 8 g |
|---|---|
| glycerin distearate | 2 |
| γ-linolenic acid | 3 |
| octaethylene glycol dodecyl ether | 1.0 |

Example 3

A mixture comprising the components 1 of the formulation which will be described below was heated to obtain a homogeneous solution. The component 2 was added to the obtained solution under stirring to prepare a W/O emulsion. This emulsion was added to 10 g of a 10% aqueous solution of polyethylene hardened castor oil (HCO-60; a product of Nikko Chemicals Co., Ltd.) under stirring. The obtained liposome was a stable and minute particle having an average size of 0.2 micron.

Formulation:

| | | |
|---|---|---|
| ① | purified egg yolk lecithin | 8 g |
| | glycerin distearate | 2 |
| | γ-linolenic acid | 5 |
| ② | purified water | 5 |

Example 4

Formulation:

| purified hydrogenated soybean lecithin | 4 g |
|---|---|
| HCO-60 (polyoxyethylene hydrogenated castor oil) | 0.4 |
| vitamin E | 1 |
| water | 94.6 |

The above components were homogeneously dispersed and subjected to ultrasonic treatment at 80°C for about 10 minutes. The obtained liposome was a stable and minute particle having an average size of 0.15 μm.

Example 5

Formulation:

4

|   |   |   |
|---|---|---|
| ① | purified soybean lecithin | 2 g |
|   | Decaglyn 1-L (decaglyceryl monolaurate) | 0.4 |
|   | vitamin A | 0.1 |
|   | ethanol | 5 |
| ② | water | 92.5 |

The components 1 were dissolved by stirring to obtain a solution. This solution was injected into the component 2 with a syringe. The obtained liposome was stable and had an average particle size of 0.2 μm.

Example 6

Formulation:

|   |   |
|---|---|
| purified hydrogenated soybean lecithin | 10 g |
| HCO-60 | 0.5 |
| vitamin A | 1 |
| heptane | 50 |

The above components were stirred under heating to obtain a homogeneous solution. The obtained solution was spray-dried to remove the heptane, thus giving a powdery sample.

2 g of this sample was added to 100 g of a 20% aqueous solution of ethanol. The obtained mixture was subjected to ultrasonic treatment at 65°C for about 5 minutes to obtain a stable liposome having an average particle size of 0.1 μm.

Example 7

Formulation:

| purified hydrogenated egg yolk lecithin | 7 g |
|---|---|
| HCO-60 | 0.5 |
| cholesterol | 2 |
| dicetyl phosphate | 1 |
| γ-linolenic acid | 2 |
| toluene | 50 |

The above components were stirred under heating to obtain a homogeneous solution. The obtained solution was spray-dried to remove the toluene, thus obtaining a powdery sample.

5 g of this sample was added to 100 cc of water. The obtained mixture was heated and stirred with a homomixer (8000 rpm) at 70°C for 5 minutes to obtain a stable liposome having an average particle size of 0.3 μm.

Example 8

Formulation:

| purified soybean lecithin | 5 g |
|---|---|
| MYS-40 (polyoxyethylene stearate) | 0.5 |
| casein | 0.5 |
| vitamin E | 1 |
| chloroform | 100 |

The above components were homogeneously dissolved and heated in an evaporator to remove the chloroform, thus obtaining a transparent thin film.

200 cc of water was added to this film. The obtained mixture was stirred with a homomixer (8000 rpm) for 5 minutes to obtain a stable liposome having an average particle size of 0.2 μm.

Example 9

Formulation:

| | |
|---|---|
| purified hydrogenated soybean lecithin | 4 g |
| pluronic (polyoxyethylene polyoxypropylene glycol ether) | 0.4 |
| soybean oil | 4 |
| water | 91.6 |

The above components were stirred under heating and further stirred with a homomixer (8000 rpm) at 80°C for 10 minutes to obtain a stable liposome having an average particle size of 0.3 $\mu$m.

Example 10

Formulation:

| | | |
|---|---|---|
| ① { | cholesterol | 3 g |
| | soybean oil | 10 |
| ② { | sodium salicylate | 2 |
| | water | 85 |

The above components 1 were homogeneously dissolved to obtain a solution. The above components 2 were added to the solution under stirring to prepare a W/O emulsion. A mixture having the undermentioned formulation containing this W/O emulsion was heated to 70°C under stirring and stirred with a homomixer - (8000 rpm) for 10 minutes to obtain a stable liposome having an average particle size of 0.2 $\mu$m.

Formulation:

| | |
|---|---|
| purified hydrogenated soybean lecithin | 5 g |
| HCO-60 | 0.5 |
| vitamin A | 0.1 |
| W/O emulsion | 10 |
| water | 84.4 |

Example 11

Formulation:

7

$$
① \begin{cases}
\text{diglyceryl monooleate} & 4 \text{ g} \\
\text{purified soybean lecithin} & 1 \\
\text{glycerin} & 4 \\
\text{squalane} & 10
\end{cases}
$$

$$
② \begin{cases}
\text{dipotassium glycyrrhizate} & 0.2 \\
\text{water} & 80.8
\end{cases}
$$

The above components 1 were homogeneously dispersed to obtain a dispersion. The components 2 were added to the dispersion under stirring to prepare a W/O emulsion. A mixture having the undermentioned formulation containing the W/O emulsion was heated to 70°C under stirring and treated in an ultrasonic homogenizer for 5 minutes to obtain a stable liposome having an average particle size of 0.1 μm.

Formulation:

| | |
|---|---|
| hydrogenated soybean lecithin | 5 g |
| Decaglyn 1-M (decaglyceryl monomyristate) | 1 |
| vitamin E | 1 |
| W/O emulsion | 10 |
| water | 83 |

Example 12

Formulation:

| | |
|---|---|
| purified egg yolk lecithin | 2 g |
| HCO-50 (polyoxyethylene hydrogenated methionine castor oil) | 0.2 |
| | 0.2 |
| vitamin $D_3$ | 0.02 |
| water | 97.58 |

The above components were mixed under heating and stirred with a colloid mill at 70°C to prepare a liposome. The obtained liposome was stable and had an average particle size of 0.15 μm.

Example 13

Formulation:

| | |
|---|---|
| purified hydrogenated soybean lecithin | 3 g |
| BC-15 (polyoxyethylene cetyl ether) | 0.2 |
| arginine | 0.3 |
| $\gamma$ -linolenic acid | 2 |
| water | 94.5 |

The above components were mixed under heating and treated in a homogenizer at 70°C to prepare a liposome. This liposome was stable and had an average particle size of 0.2 μm.

Example 14

Formulation:

| | |
|---|---|
| purified hydrogenated egg yolk lecithin | 10 g |
| octaethylene glycol dodecyl ether | 1 |
| castor oil | 1 |
| indomethacin or indometacin | 2 |
| heptane | 50 |

The above components were stirred under heating to obtain a homogeneous solution. The heptane was removed from the obtained solution by spontaneous drying to obtain an aggregate. This aggregate was pulverized into a powder. 100 cc of water was added to 5 g of this powder. The obtianed mixture was stirred with a homomixer (8000 rpm) at 80°C for 10 minutes to obtain a stable liposome having an average particle size of 0.2 μm.

Example 15

Formulation:

| | |
|---|---|
| purified hydrogenated soybean lecithin | 5 g |
| Decaglyn 1-S (decaglyceryl monostearate) | 0.5 |
| glycerin distearate | 0.5 |
| glycerin | 10 |

The above components were dissolved by heating to prepare a solution. 200 cc of water was added to the solution. The obtained mixture was stirred with a homomixer (8000 rpm) at 80°C for 10 minutes to obtain a stable liposome having an average particle size of 0.2 μm.

Example 16

Formulation:

| | |
|---|---|
| dipalmitoyl phophatidyl choline | 5 g |
| HCO-60 | 0.7 |
| phytosterol | 0.5 |
| ubidecarenone | 1 |
| hexane | 50 |

The above components were homogeneously dissolved and spray-dried to remove the hexane, thus obtaining a powdery sample.

100 cc of water was added to 5 g of this sample. The obtained mixture was subjected to ultrasonic treatment at 60°C for about 5 minutes to obtain a stable liposome having an average particle size of about 0.1 μm.

Example 17

Formulation:

| | |
|---|---|
| purified hydrogenated soybean lecithin | 4 g |
| decaglyn 1-0 (decaglyceryl monooleate) | 0.6 |
| MGS (glycerin monostearate) | 0.4 |
| vitamin E | 1 |
| water | 94 |

The above components were heated to 80°C under stirring and stirred with a homomixer (8000 rpm) for 10 minutes to obtain a stable liposome having an average particle size of 0.2 μm.

## Claims

1. A process for the preparation of a liposome which comprises mixing water with both a phospholipid and a hydrophilic non-ionic surfactant.

2. A process as claimed in claim 1 in which the water is concurrently mixed with the phodpholipid and the surfactant.

3. A process as claimed in claims 1 or 2 in which a powder comprising both a phospholipid and the surfactant is dispersed in water.

4. A process as claimed in claim 1 or 2 in which a homogenous liquid containing both the phospholipid and the surfactant is dispersed in water.

5. A process as claimed in claim 1 or 2, in which an aqueous solution of the surfactant is mixed with a preformed water in oil emulsion containing the phospholipid in the oily phase.

6. A process as claimed in any of the preceding claims in which the mixture or dispersion so formed is subjected to high speed mechanical agitation.

7. A process as claimed in any of the preceding claims in which a stabilizer for the liposome is also added.

8. A process as claimed in any of the preceding claims in which the mixing of the water with the phospholipid and the surfactant is effected in the presence of a dispersing agent.

9. A process as claimed in any of the preceding claims in which 1 to 15% by weight of the surfactant based on the weight of phospholipid is used.

10. A process as claimed in any of the preceding claims in which the phospholipid is a hydrogenated phospholipid.

11. A process as claimed in any of the preceding claims in which a desired component to be enveloped by the lyposome is present during the formation of the lyposome.

12. A process as claimed in claim 11 in which the desired component is a drug.